(19) **Europäisches Patentamt / European Patent Office / Office européen des brevets**

(11) **EP 4 574 042 A1**

(12) **EUROPEAN PATENT APPLICATION**

(43) Date of publication:
25.06.2025   Bulletin 2025/26

(51) International Patent Classification (IPC):
*A61B 5/22* (2006.01)      *A61H 3/00* (2006.01)
*B25J 9/00* (2006.01)

(21) Application number: 23383352.4

(22) Date of filing: 21.12.2023

(52) Cooperative Patent Classification (CPC):
B25J 9/0006; A61B 5/224

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(71) Applicant: Fundación Tecnalia Research and Innovation
20009 Donostia-San Sebastian (ES)

(72) Inventors:
• MCINTYRE, Joseph
20009 Donostia - San Sebastian (ES)

• BENGOETXEA ARRESE, Ana
Bruxelles (BE)
• DOMÍNGUEZ GARCÍA, Alfonso
20009 Donostia - San Sebastian (ES)
• SERENA FAORO, Vitalie
Bruxelles (BE)
• JUNG, Je Hyung
20009 Donostia - San Sebastian (ES)

(74) Representative: Balder IP Law, S.L.
Paseo de la Castellana 93
5ª planta
28046 Madrid (ES)

(54) **SYSTEM AND METHOD FOR EVALUATING EXOSKELETONS**

(57)     The invention relates to a system and a methos for evaluating exoskeletons. The system comprises: a testbed including a workspace configured to allow a user to perform a predetermined task, wherein the testbed is fitted with sensors to measure forces exerted by the user to complete the predetermined task, and processing means to process data provided by the sensors, to determine the mechanical action expended by the user to complete the task, and to evaluate whether the predefined task has been completed on two occasions in the testbed by the user with the same amount of mechanical action. The system has means for measuring physiological parameters of the user, in order to evaluate the level of fatigue experienced by the user after completing the predefined task in the testbed. The testbeds ensures that fatigue evaluations are performed and compared only after the worker has performed the same amount of activity with and without the target exoskeleton.

FIG.1

**EP 4 574 042 A1**

## Description

## TECHNICAL FIELD

The present invention relates to a system and a methodology for evaluating exoskeletons, in order to assist industrial actors to select an exoskeleton which might aid their workers while carrying out a particular task.

An object of the invention is to provide a methodology for evaluating exoskeletons, which is reliable and simple, and which can be applied to a wide variety of working environments and to a broad range of commercially available exoskeletons, for assessing whether the use of a particular exoskeleton could lead to a decrease in muscular, metabolic and central fatigue at the end of a workday, increase productivity and facilitate recovery.

## STATE OF THE ART

Despite enormous gains in the areas of robotics and automation, construction and manufacturing are still labour-intensive undertakings. Millions of workers are employed worldwide to assemble and deploy the many buildings and machines on which our daily lives rely. And while human workers provide versatility and robustness unmatched by any machine (robot or otherwise), humans remain intrinsically fragile with respect to the physical demands of manual labour. Not only must one assure the basic physical safety of human workers in perilous environments, even relatively tame tasks that involve repetition or awkward physical gestures can lead to incapacitating injuries known collectively as musculoskeletal disorders (MSDs). Treatment and compensation for work-loss due to these injuries places a significant burden on manufacturers and healthcare systems. Changing demographics and an ageing society compound these costs to society, with increased vulnerability to MSDs in an older workforce and increased need to protect workers in physically strenuous caregiving roles for the elderly.

In recent years, exoskeletons have been put forward as the solution to all our problems associated with manual occupations. By transferring the loads of physically demanding jobs to these mechatronic devices that follow and reinforce our every move, it was thought that we as workers would become bionic super-humans, impervious to the stresses and strains of manual labour. But the promise of effortless labour thanks to exoskeletons has yet to be realized in the real world. Beyond the fact that it is technologically very challenging to create an effective exoskeleton adapted to real-world conditions, industrial actors are reluctant to invest in exoskeleton technologies for their workers because they do not have reliable means to determine if and how a particular exoskeleton might benefit their workers, nor if there will be a net return on investment if one adopts these technologies.

Integrating exoskeletons into occupational settings necessitates rigorous evaluations of their ergonomic impact. This involves assessing their effects on their ability to mitigate workplace injuries and fatigue. Some known ergonomic tools offer structured approaches to evaluating the biomechanical aspects of workplace ergonomics and to estimate the associated risks of musculoskeletal injury. Some of these tools can be used to objectively evaluate risk factors based on easily assessed, observable task characteristics such as body postures, lifting heights and load magnitudes (weights) have been validated by decades of research and experimentation based on physiological measurements and biomechanical modelling of stresses and strains on the human tissues during a variety of load-bearing tasks. But the conventional assessment tools cannot be directly applied to evaluations when exoskeletons are involved.

Given the relative infancy of exoskeleton technologies, ergonomics assessment methodologies that account for the actions of the exoskeletons in terms of reducing or transferring loads on different parts of the body are much less evolved and have not yet achieved the same levels of simplicity and acceptance that the more conventional tools enjoy.

Currently, relatively sophisticated physiological measurements are required to assess exoskeleton efficacy. By employing technologies such as motion capture systems and electromyography, researchers in the lab can analyze how exoskeletons influence the movements and muscle activity of users. These quantitative measurements provide valuable data on the biomechanical effects of exoskeletons, helping to determine their impact on worker ergonomics, but the bridge to real-world conditions is not always evident.

The majority of the scientific articles focus on the impact on the muscle groups which are intended to be supported by the assistant devices. Due to the onerous nature of such tests, they are typically conducted on limited numbers of subjects in somewhat artificial conditions. In general, this leads to the conclusion that the specific exoskeleton is beneficial within the scope of the study. But the potential negative aspects, for example the biomechanical load shift to other joints or muscle groups and further trade-offs, are rarely investigated. What is lacking, therefore, are reliable and convenient methodologies for assessing whether the use of an exoskeleton in a particular and realistic work environment will lead to a decrease in muscular and central fatigue at the end of the workday.

Current state-of-the-art for exoskeleton evaluation can therefore be divided into two main categories:

    1. elaborate, cumbersome and time-consuming testing procedures invoking direct measurement of physiological parameters such as cardiovascular load, oxygen consumption and muscle activation;

2. worksheets or formulas based on relatively simple measurements and observations (load weight, postures) that evaluate physical loads on the body but that do not evaluate fatigue *per se* and that need to be redesigned and validated for each new exoskeleton technology.

Therefore, there is a need in this technical field for a methodology that can reliably and easily evaluate a wide range of commercially available exoskeletons, providing an accurate assessment of the reduction in muscular and central fatigue achieved by the use of a particular exoskeleton.

## DESCRIPTION OF THE INVENTION

The invention is defined in the attached independent claims, and satisfactorily overcomes the above-described shortcomings of the prior art, by the provision of a methodology built upon evaluations using instrumented testbeds designed to replicate real-world working conditions of a manufacturing tasks, and to ensure reproducible testing conditions for evaluating exoskeletons. Furthermore, a testing regime is carried out of pre- and post-tests specifically designed to quantify the level of fatigue experienced by a human user when performing a simulated work tasks with or without an exoskeleton.

The methodology of the invention is capable of quantifying central and muscular fatigue, based on simple tests that do not involve sophisticated physiological measurements such as electromyography or ($VO_2$) oxygen consumption, while the testbeds automate the processing of measuring the worker's activity during the test trials, to ensure that fatigue evaluations are performed and compared only after the worker has performed the same amount of activity with and without the target exoskeleton.

More specifically, an aspect of the invention refers to a system for evaluating exoskeletons, which comprises a testbed including at least one workspace configured to allow a user to perform a predetermined task involving exercising mechanical actions. The testbed is fitted with a plurality of sensors arranged to capture data relative to the actions performed by the user, and to measure forces exerted by the user to complete the predetermined task.

The system further comprises processing means adapted to process data provided by the sensors, to determine the mechanical action expended by the user to complete the task. The processing means are further adapted to evaluate whether the predefined task has been completed on two occasions in the testbed by the user with the same amount of mechanical action.

Preferably, the processing means are embodied as a computer programmed to operate in two modes, namely a first mode wherein the test subject works for a fixed amount of time and the computed mechanical action is used to normalize the fatigue score. In a second mode, the testbed computes the cumulative mechanical action in real time and the subject stops when a threshold has been reached.

The system further comprises means for measuring and/or testing physiological parameters of the user, in order to evaluate the level of muscular and/or central fatigue experienced by the user after completing the predefined task in the testbed.

Additionally, the system may include means for comparing the muscular and/or central fatigue experienced by the user after completing the predetermined task in two occasions with the same amount of mechanical action, one occasion without using an exoskeleton to be tested, and another one with the exoskeleton to be tested, so that the degree of assistance of the exoskeleton in reducing the fatigue of the user, can be evaluated. The means for comparing the muscular and/or central fatigue, may be implemented by a programmable device.

The testbed is configured to realistically simulate several building, manufacturing or handling tasks in a sensorized environment, for example requiring the use of tools above the height of a person's head in a standing position. The methodology is based on performing a set of physiological tests before and after defined periods of activity in the target manufacturing or construction task. The physiological tests are designed to detect increases or decreases in the level of fatigue exhibited by the test subject when using an exoskeleton versus performing the same activities without it.

The testbed has a vertical workspace and a horizontal workspace with respect to ground, wherein the horizontal workspace is placed above the vertical workspace. Preferably, the size of the vertical and horizontal workspaces is adjustable.

The sensors provide a critical independent measurement that the amount of "mechanical action" performed while performing the task are the same, within certain margins, between the epochs where the exoskeleton is employed and epochs where it is not.

The term "mechanical action" requires a formal definition in this context. Typically, one might envisage comparing epochs where the same mechanical work ($W = F \times d$) is performed, meaning that the same amount of energy has been transferred between the worker or worker + exoskeleton and the environment. However, applying forces against a rigid, immovable constraint generates no work, as there is no displacement. Nevertheless, a human muscle or a linear motor will consume energy when exerting a force against an immovable constraint: metabolic energy for the muscle and electrical energy (Watt hours) for the motor. In the case of tasks typically performed in overhead conditions, such as painting, sanding, plastering or screwing, we consider the integral of the force over time (formally known as the total impulse) as indicative of the mechanical action performed by the subject on the environment when performing such tasks. If the user exhibits lesser or greater fatigue after several epochs of performing the defined task for the same total impulse when using the exoskeleton, versus not using the exoskeleton, the exoskeleton will be judged to be a benefit or a disadvantage for the user.

Force sensors are therefore integrated into the testbed to allow automated quantification of the amount of mechanical action performed against the testbed structure, to provide a reference for interpreting the physiological measurements.

In a preferred embodiment, the sensors are force sensors arranged in the vertical and horizontal workspaces, to measure forces applied by the user to the workspace during the completion of the same predetermined task.

Preferably, the system has four sensors arranged on the same plane in the vertical workspace, and/or four sensors arranged on the same plane on the horizontal workspace. The system includes processing means adapted to determine the number of actions involving exercising a force performed by the user in the workspace, the location of each action on that plane, and the force applied by the user in each action, based on measurements of the force sensors.

The means for measuring and/or testing physiological parameters of the user, comprises means for measuring or testing at least one of the following physiological parameters: blood pressure, blood pressure recovery, respiratory rate, respiratory rate recovery, heart rate, heart rate recovery, heart rate variability, lactate accumulation, perceived effort, perceived dyspnea, maximal force exerted, exerted-force interoception test or a dual-task time-up-and-go test.

Another aspect of the invention refers to a method for evaluating exoskeletons comprising the following steps:

    providing a sensorised testbed having at least one workspace adapted so that a user can perform a predetermined task, the predetermined task involving making mechanical actions,

    capturing data from the sensors in the testbed relative to the actions and forces exerted by the user during the completion of the predetermined task, without using an exoskeleton,

    measuring and/or testing physiological parameters from the user before and after completing the predetermined task without using an exoskeleton, and processing the measured physiological parameters to quantify the level of muscular and/or central fatigue experienced by the user after completing the predefined task without using an exoskeleton,

    capturing data from the sensors in the testbed relative to the same actions and same forces exerted by the user during the completion of the same predetermined task in the testbed, using an exoskeleton,

    measuring and/or testing physiological parameters from the user before and after completing the predetermined task using the exoskeleton, and processing the measured physiological parameters to quantify the level of muscular and/or central fatigue experienced by the user after completing the predefined task using an exoskeleton, and

    comparing the quantified levels of muscular and/or central fatigue experienced by the user using an exoskeleton and without it, such that the degree of assistance of the exoskeleton in reducing the fatigue of the user, can be evaluated.

In the methodology of the invention, the testbed ensures that the epochs of activity be the same for tests performed with and without the exoskeleton, in order to ensure that any changes in fatigue level induced by the exoskeleton can be attributed to the effects of the exoskeleton itself, and not to simple variations in the amount of useful task activity performed between the two conditions.

Preferably, the method further comprises processing the data provided by the sensors to determine whether the step of involving making a mechanical action expended by the user to complete the predetermined task with and without exoskeleton, has been substantially the same.

The step of measuring and/or testing physiological parameters, comprises measuring or testing at least one of the following physiological parameters: blood pressure, blood pressure recovery, respiratory rate, respiratory rate recovery, heart rate, heart rate recovery, heart rate variability, lactate accumulation, perceived effort, perceived dyspnea, maximal force exerted, exerted-force interoception test or a dual-task time-up-and-go test.

**BRIEF DESCRIPTION OF THE DRAWINGS**

To complete the description and in order to provide a better understanding of the invention, a set of drawings is provided. These drawings form an integral part of the description and illustrate embodiments of the invention, which should not be interpreted as restricting the scope of the invention, but just as examples of how the invention can be carried out. The drawings comprise the following figures:

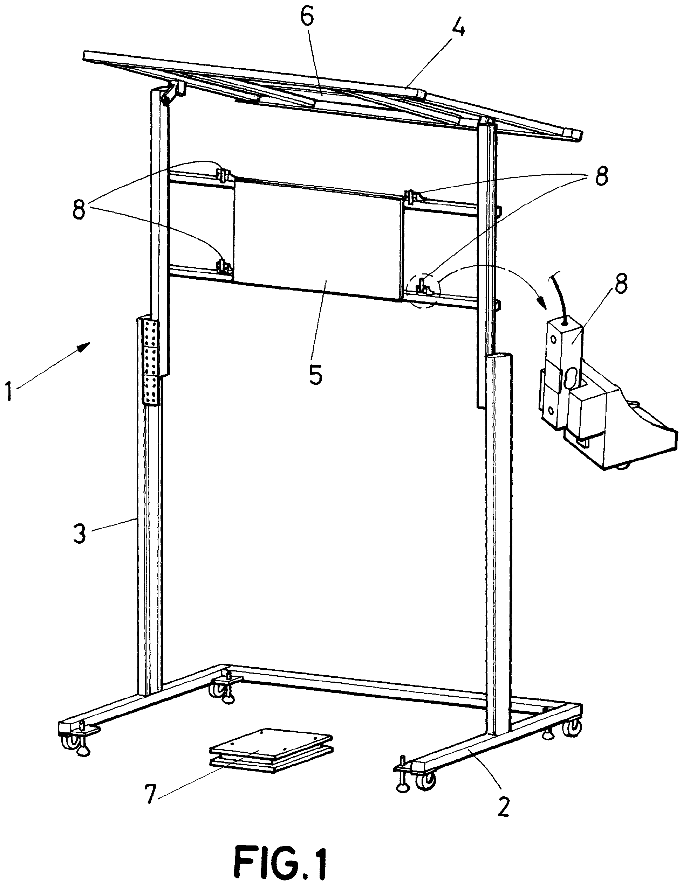

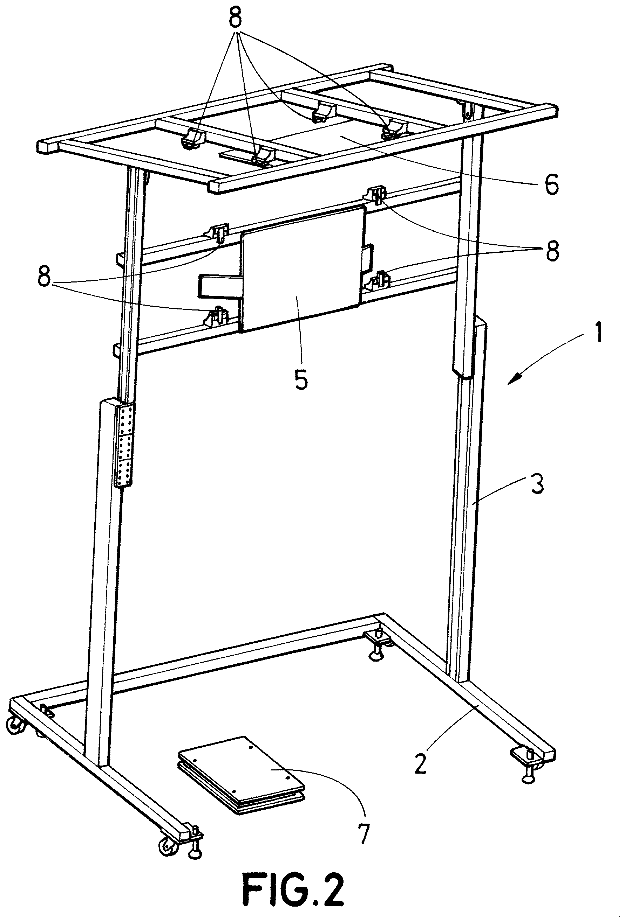

Figures 1 and 2.- show two perspective views of a testbed according to the invention.

**PREFERRED EMBODIMENTS OF THE INVENTION**

As shown in **Figures 1** and **2**, the testbed (1) is formed by a horizontal frame (2) at the bottom part thereof fitted with wheels, a vertical structure (3) attached to the horizontal frame (2), and an overhead frame (4) attached to a top part of the vertical structure (3). A vertical workspace (5) is formed in the vertical structure (3), and a horizontal workspace (6) is formed in the overhead frame (4). A force plate (7) might be used to detect the position and ground reaction force of a test subject while performing the test.

To measure the interaction force occurring while performing work tasks using the testbed, four of 50 kg and 1-axis loadcells (8) are placed on either the horizontal or the vertical workspaces as shown in **Figure 2**. The use of four loadcells (8) allows measuring not only the magnitude of the force but also the point to which the force applies.

The position can be estimated from the load cell signals using the relation,

$$x = width \cdot (f3+f4)/(f1+f2+f3+f4)$$

$$y = height \cdot (f1+f4)/(f1+f2+f3+f4)$$

where:

f1 is the signal of the upper left load cell,
f2 is the signal of the lower left load cell,
f3 is the signal of the lower right load cell and
f4 is the signal of the upper right load cell.

It might be required that the defined activities are to be performed either horizontally or vertically. Hence the overhead testbed has a configurable structure and mechanism to facilitate the simulation of those activities. The specifications of the overhead testbed are as follows:

- the testbed provides a workspace for the tasks that are performed in both horizontal and vertical planes;
- the size of the workspace for the tasks is configurable;
- the height of the horizontal area of the testbed is adjustable;
- the testbed measures the interaction forces that occur while performing the tasks;
- the testbed is movable;
- the orientation of the horizontal workspace is changeable between 0° and 15°;
- the horizontal workspace can be moved in either forward or backward directions.

The testbed might be built with versatile aluminum profiles, thus allowing easy reconfiguration of both vertical and horizontal workspaces. For example, two aluminum profiles in the vertical workspace can be easily placed at other locations, resulting in a quick decrease or increase in the workspace. The horizontal workspace is 2m x 2m while the vertical workspace is 2.1 m x (1m ~ 3.3m).

## 1.- Physiological Tests.

The testbed described above are coupled with a testing regime of pre- and post-tests specifically designed to quantify the level of fatigue experienced by test subjects when performing the simulated work tasks with or without an exoskeleton. The methodologies quantify central and muscular fatigue based on simple tests that do not involve sophisticated physiological measurements such as electromyography or oxygen consumption ($VO_2$). The tests include resting metabolism, cardiorespiratory function assessment, perceived effort and dyspnea questionnaires. The tests are repeated at the same time of the day for each subject, once with and once without use of an exoskeleton, with "normal" smoking/caffeine intake in the 24h before testing.

### 1.1.- Initial Tests.

The clinical examination starts with an anamnesis, based on the relevant medical history and the subject's sports practice. Additional measurements include systolic and diastolic systemic blood pressure (sphygmo-manometry), heart rate (HR) and arterial $O_2$ saturation ($SpO_2$) (finger pulse oximetry) are measured at rest in a seated position. After a large period of quietness, ECG is recorded for heart rate (HR) and HR variability (HRV) analysis during 7 min (frequency and temporally analyses can be performed). Those measurements are associated with resting ventilation (breathing frequency and tidal volume) recordings.

### 1.2.- Cardiorespiratory Tests.

#### 1.2.1.- Blood Pressure.

Just after each working epoch in a sitting position, systemic and diastolic blood pressure are measured and compared to the resting values. A smaller change of blood pressure after working with the exoskeleton versus without is indicative that the exoskeleton provides a net benefit in terms of reducing the physiological effort required by the task.

#### 1.2.2.- Blood Pressure Recovery.

Blood pressure is recorded at fixed intervals after termination of the working epoch. The time required to return to pre-test BP levels is an indication of the level of cardio-vascular demand and fatigue during the work epoch. A shorter time to recovery after working with the exoskeleton versus without is indicative that the exoskeleton provides a net benefit in terms of reducing the physiological effort required by the task.

#### 1.2.3.- Respiratory Rate.

Respiration rate is measured at the number of breaths per minute immediately after termination of the work epoch. Lower respiration rate after working with the exoskeleton versus without is indicative that the exoskeleton provides a net benefit in terms of reducing the physiological effort required by the task.

### 1.2.4.- Respiratory Rate Recovery.

Respiration rate is recorded at fixed intervals after termination of the working epoch. The time required to return to pre-test BP levels is an indication of the level of respiratory demand and fatigue during the work epoch. A shorter time to recovery after working with the exoskeleton versus without is indicative that the exoskeleton provides a net benefit in terms of reducing the physiological effort required by the task.

### 1.2.5.- Heart Rate.

Heart rate is measured in beats per minute immediately after termination of the work epoch. Lower heart rate after working with the exoskeleton versus without is indicative that the exoskeleton provides a net benefit in terms of reducing the physiological effort required by the task.

### 1.2.6.- Heart Rate Recovery.

Heart rate is recorded at fixed intervals after termination of the working epoch. The time required to return to pre-test heart rate levels is an indication of the level of cardiovascular demand and fatigue during the work epoch. A shorter time to recovery after working with the exoskeleton versus without is indicative that the exoskeleton provides a net benefit in terms of reducing the physiological effort required by the task.

### 1.2.7.- Heart Rate Variability.

Heart rate variability (HRV) is the fluctuation in the time intervals between adjacent heartbeats under control of the autonomic nervous system. Heart rate is measured with an ECG monitoring device. Higher HRV is associated with lower stress, higher adaptability, improved cognition and increased performance. Higher HRV after working with the exoskeleton is indicative that the exoskeleton provides a net benefit in terms of reducing the physiological effort required by the task and recovery.

### 1.2.8.-Lactate Accumulation.

Lactate accumulation between work epochs informs about the metabolic substrate used by the subject during the task and therefore describes the type of muscle fibre recruited during the task. Comparing with and without exoskeleton lactate accumulation is used to quantify the degree of anaerobic metabolic fatigue. Lower blood lactate accumulation after working with the exoskeleton is indicative that the exoskeleton provides a net benefit in terms of lower intensity of work, less demand on anaerobic metabolism, less fatigue accumulation and lower muscle fiber 2 recruitement (generating great forces but highly fatigable).

### 1.2.9.- Perceived Fatigue.

Perceived effort is reported on a visual analog scale after each working epoch. Lower score ratings are indicative of a reduced effort perceived by the subject.

### 1.2.10.- Dyspnea.

Dyspnea (shortness of breath) is estimated by the subject using a modified Borg scale. Lower scores are indicative of a reduced respiratory effort and perceived exertion.

### 1.3.- Muscular and Central Fatigue

### 1.3.1. Maximal force exerted

The subject exerts a force against a dynamometer with the arm (or leg) to achieve a specified target value that is the subject's maximum possible exerted force and maintain it during 3 seconds. After 20-second rest periods, the subject is requested to reproduce the target force 3 times. A reduction of the decrease of maximal force exerted after working with the exoskeleton versus without is indicative that the exoskeleton provides a net benefit in terms of reducing the muscular effort required by the task.

### 1.3.1.-Interoceptive Evaluation

The subject exerts a force against a dynamometer with the arm or leg to achieve a specified target value that is the subject's maximum possible exerted force and maintain it during 3 seconds. The subject is told the measured value of the initial exerted force to be used as a reference. After 20-second rest periods, the subject is requested to reproduce the target force 3 times, without feedback from the dynamometer. Each time the subject is requested to estimate the force that he or she produced (precision score), to be compared with the actual force produced (actual score) and at the same time he or she has to determined his or her degree of confidence with an visual analog scale (1-10) (confidence score). Precision: determining whether the new maximum force is more or less intense than the previous one makes it possible to evaluate the subject's ability to perceive their maximum force as a function of fatigue. The subject's response is compared to the actual quantitative value of the signal intensity, resulting in a certain number of correct or false responses. This process aims to quantitatively measure the interoceptive capacity of an individual on the evolution of their maximum force as a function of fatigue and the use or not of an exoskeleton. This interoceptive capacity should not be modified unless cognitive fatigue and/or a priori on the use of an exoskeleton are present. Confidence: The aim is to establish to what extent participants are able to correctly evaluate their own performance. We evaluate whether subjects' confidence is a reliable indicator of their actual performance in a precision task. For example, if a subject has high confidence in an inaccurate response, this indicates poor metacognitive calibration and conversely, if a participant is confident in a correct response, this indicates good metacognitive calibration. This process makes it possible to establish a metacognitive index (degree of calibration) between confidence and the precision task. The degree of association between accuracy and confidence is considered a quantitative measure of metacognition. This metacognition capacity should not be modified unless cognitive fatigue and/or a priori on the use of an exoskeleton are present.

1.3.2.- Dual-Task Time-Up-And-Go (TUGd)

The time-up-and-go (TUG) test consists of getting up from a chair, walking 3m, turning around a point and coming back to sit down. The goal is to do the task as quickly as possible, without running. Time Up and Go dual-task version (TUGd) consist in realizing the same task by combining a cognitive task, in our case subtracting 3 by 3 from a random number. In this case, in addition to take into account the time for performing the motor task, this test takes into account the calculation errors made during the test. While the simple TUG evaluates motor skills for dynamic balance and has been proven to be able to identify risk of falls, TUGd assesses the capacity to share attention between a cognitive and a motor task, and it is an indirect way to look for cognitive or mental fatigue. If calculation errors are present and/or if the walking time is increased more than 10%, this indicates the presence of mental (or central) fatigue.

**Claims**

1. A system for evaluating exoskeletons, comprising:

    a testbed including at least one workspace configured to allow a user to perform a predetermined task involving exercising mechanical actions,
    wherein the testbed is fitted with a plurality of sensors arranged to capture data relative to the actions performed by the user, and to measure forces exerted by the user to complete the predetermined task,
    processing means adapted to process data provided by the sensors, to determine the mechanical action expended by the user to complete the task, the processing means further adapted to evaluate whether the predefined task has been completed on two occasions in the testbed by the user with the same amount of mechanical action, and
    means for measuring and/or testing physiological parameters of the user, in order to evaluate the level of muscular and/or central fatigue experienced by the user after completing the predefined task in the testbed.

2. System according to claim 1, further comprising means for comparing the muscular and/or central fatigue experienced by the user after completing the predetermined task in two occasions with the same amount of mechanical action, one occasion without using an exoskeleton to be tested, and another one with the exoskeleton to be tested, so that the degree of assistance of the exoskeleton in reducing the fatigue of the user, can be evaluated.

3. System according to claim 1, wherein the testbed has a vertical workspace and a horizontal workspace with respect to ground, wherein the horizontal workspace is placed above the vertical workspace, and wherein the size of the vertical and horizontal workspaces is adjustable.

4. System according to any of the preceding claims, wherein the sensors are force sensors arranged in the vertical and horizontal workspaces, to measure forces applied by the user to the workspace during the completion of the same predetermined task.

5. System according to claim 4, comprising four sensors arranged on the same plane in the vertical workspace, and/or four sensors arranged on the same plane on the horizontal workspace, the system further comprising processing means adapted to determine the number of actions involving exercising a force performed by the user in the workspace, the location of each action on that plane, and the force applied by the user in each action, based on measurements of the force sensors.

6. System according to any of the preceding claims, wherein the means for measuring and/or testing physiological parameters of the user, comprises means for measuring or testing at least one of the following physiological parameters: blood pressure, blood pressure recovery, respiratory rate, respiratory rate recovery, heart rate, heart rate recovery, heart rate variability, lactate accumulation, perceived effort and dyspnea, maximal exerted force, exerted-force interoception test or a dual-task time-up-and-go test.

7. Method for evaluating exoskeletons, comprising:

providing a sensorised testbed having at least one workspace adapted so that a user can perform a predetermined task involving making mechanical actions,

capturing data from the sensors in the testbed relative to the actions and forces exerted by the user during the completion of the predetermined task, without using an exoskeleton,

measuring and/or testing physiological parameters from the user before and after completing the predetermined task without using an exoskeleton, and processing the measured physiological parameters to quantify the level of muscular and/or central fatigue experienced by the user after completing the predefined task without using an exoskeleton,

capturing data from the sensors in the testbed relative to the same actions and same forces exerted by the user during the completion of the same predetermined task in the testbed, using an exoskeleton,

measuring and/or testing physiological parameters from the user before and after completing the predetermined task using the exoskeleton, and processing the measured physiological parameters to quantify the level of muscular and/or central fatigue experienced by the user after completing the predefined task using an exoskeleton, and

comparing the quantified levels of muscular and/or central fatigue experienced by the user using an exoskeleton and without it, such that the degree of assistance of the exoskeleton in reducing the fatigue of the user, can be evaluated.

8. Method according to claim 7, further comprising processing the data provided by the sensors to determine whether the step of involving making a mechanical action expended by the user to complete the predetermined task with and without exoskeleton, has been substantially the same.

9. Method according to claim 7 or 8, wherein the step of measuring and/or testing physiological parameters, comprises measuring or testing at least one of the following physiological parameters: blood pressure, blood pressure recovery, respiratory rate, respiratory rate recovery, heart rate, heart rate recovery, heart rate variability, lactate accumulation, perceived effort and dyspnea, maximal exerted force, exerted-force interoception test or a dual-task time-up-and-go test.

FIG.1

# FIG.2

**EUROPEAN SEARCH REPORT**

Europäisches
Patentamt
European
Patent Office
Office européen
des brevets

Application Number

EP 23 38 3352

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | ANN VIRTS ET AL: "A peg-in-hole test and analysis method for exoskeleton evaluation", NIST, NATIONAL INSTITUTE OF STANDARDS AND TECHNOLOGY (NIST) , 7 March 2022 (2022-03-07), pages 1-31, XP061078906, DOI: 10.6028/NIST.TN.2208 Retrieved from the Internet: URL:https://nvlpubs.nist.gov/nistpubs/Tech nicalNotes/NIST.TN.2208.pdf [retrieved on 2022-03-07] | 1-4,6-9 | INV. A61B5/22 A61H3/00 B25J9/00 |
| A | * abstract; figures 1,2 * <br> * page 2, paragraphs 4,5 * <br> * page 4, paragraphs 2,4 * <br> * page 7, paragraph 5-7 * <br> * page 10, paragraph 4 - page 11, paragraph 1 * <br> * page 20, paragraph 3 * <br> * page 21, paragraph 4 * <br> * page 22, paragraph 4 * | 5 | |
| | ----- <br> -/-- | | TECHNICAL FIELDS SEARCHED (IPC) <br><br> A61B <br> B25J <br> A61H |

The present search report has been drawn up for all claims

| Place of search | Date of completion of the search | Examiner |
|---|---|---|
| Berlin | 29 May 2024 | Sleightholme, G |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

# EUROPEAN SEARCH REPORT

**Application Number**

EP 23 38 3352

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | BOSTELMAN ROGER ET AL: "Towards Standard Exoskeleton Test Methods for Load Handling", 2019 WEARABLE ROBOTICS ASSOCIATION CONFERENCE (WEARRACON), IEEE, 25 March 2019 (2019-03-25), pages 21-27, XP033554867, DOI: 10.1109/WEARRACON.2019.8719403 [retrieved on 2019-05-21] | 1-3,6-9 | |
| A | * abstract; figures 1-3 * <br> * page 22, left-hand column, paragraphs 2,3 * <br> * page 22, right-hand column, paragraphs 1,3 * <br> * page 23, left-hand column, paragraph 3 * <br> * page 23, right-hand column, paragraphs 1,2 * <br> * page 24, left-hand column, paragraph 6 - right-hand column, paragraph 1 * <br> * page 24, right-hand column, paragraph 3 * | 4,5 | |
| | ----- <br> -/-- | | **TECHNICAL FIELDS SEARCHED (IPC)** |

The present search report has been drawn up for all claims

| Place of search | Date of completion of the search | Examiner |
|---|---|---|
| Berlin | 29 May 2024 | Sleightholme, G |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 2 of 3

Europäisches
Patentamt

European
Patent Office

Office européen
des brevets

# EUROPEAN SEARCH REPORT

**Application Number**

EP 23 38 3352

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | MAURICE PAULINE ET AL: "Objective and Subjective Effects of a Passive Exoskeleton on Overhead Work", IEEE TRANSACTIONS ON NEURAL SYSTEMS AND REHABILITATION ENGINEERING, IEEE, USA, vol. 28, no. 1, 3 October 2019 (2019-10-03), pages 152-164, XP011766361, ISSN: 1534-4320, DOI: 10.1109/TNSRE.2019.2945368 [retrieved on 2020-01-07] | 1,2,6-9 | |
| A | * abstract; figure 2 *<br>* page 155, left-hand column, paragraph III.B.2 *<br>* page 156, right-hand column, paragraph III.C.5 *<br>* page 157, left-hand column, paragraphs III.D.3, 5, 7, 8 *<br>* page 157, right-hand column, paragraph III.E * | 3-5 | |
| | ----- | | **TECHNICAL FIELDS SEARCHED (IPC)** |
| A | US 2021/369542 A1 (STUART ROBERT [US] ET AL) 2 December 2021 (2021-12-02)<br>* abstract; figures 6,7 *<br>* paragraphs [0094], [0127] *<br>----- | 1-9 | |

The present search report has been drawn up for all claims

| Place of search | Date of completion of the search | Examiner |
|---|---|---|
| Berlin | 29 May 2024 | Sleightholme, G |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

........................................................................

& : member of the same patent family, corresponding document

EPO FORM 1503 03.82 (P04C01)

**EP 4 574 042 A1**

## ANNEX TO THE EUROPEAN SEARCH REPORT
## ON EUROPEAN PATENT APPLICATION NO.

EP 23 38 3352

This annex lists the patent family members relating to the patent documents cited in the above-mentioned European search report.
The members are as contained in the European Patent Office EDP file on
The European Patent Office is in no way liable for these particulars which are merely given for the purpose of information.

29-05-2024

| Patent document cited in search report | Publication date | Patent family member(s) | | Publication date |
|---|---|---|---|---|
| US 2021369542 A1 | 02-12-2021 | CA | 3179850 A1 | 02-12-2021 |
| | | CA | 3179851 A1 | 02-12-2021 |
| | | CA | 3179854 A1 | 02-12-2021 |
| | | CA | 3179855 A1 | 02-12-2021 |
| | | CA | 3179858 A1 | 02-12-2021 |
| | | CN | 115701964 A | 14-02-2023 |
| | | CN | 115715181 A | 24-02-2023 |
| | | CN | 115720514 A | 28-02-2023 |
| | | CN | 115916133 A | 04-04-2023 |
| | | CN | 115989113 A | 18-04-2023 |
| | | EP | 4157191 A1 | 05-04-2023 |
| | | EP | 4157192 A1 | 05-04-2023 |
| | | EP | 4157194 A1 | 05-04-2023 |
| | | EP | 4157195 A1 | 05-04-2023 |
| | | EP | 4157197 A1 | 05-04-2023 |
| | | IL | 298451 A | 01-01-2023 |
| | | IL | 298462 A | 01-01-2023 |
| | | IL | 298463 A | 01-01-2023 |
| | | IL | 298465 A | 01-01-2023 |
| | | IL | 298466 A | 01-01-2023 |
| | | JP | 2023528389 A | 04-07-2023 |
| | | JP | 2023528602 A | 05-07-2023 |
| | | JP | 2023528603 A | 05-07-2023 |
| | | JP | 2023528605 A | 05-07-2023 |
| | | JP | 2023530228 A | 14-07-2023 |
| | | US | 2021369540 A1 | 02-12-2021 |
| | | US | 2021369541 A1 | 02-12-2021 |
| | | US | 2021369542 A1 | 02-12-2021 |
| | | US | 2021370495 A1 | 02-12-2021 |
| | | US | 2021370496 A1 | 02-12-2021 |
| | | WO | 2021242977 A1 | 02-12-2021 |
| | | WO | 2021242980 A1 | 02-12-2021 |
| | | WO | 2021242991 A1 | 02-12-2021 |
| | | WO | 2021243056 A1 | 02-12-2021 |
| | | WO | 2021243064 A1 | 02-12-2021 |

EPO FORM P0459

For more details about this annex : see Official Journal of the European Patent Office, No. 12/82